# EUROPÄISCHE PATENTSCHRIFT

(11) **EP 0 987 549 B1**
(45) Veröffentlichungstag und Bekanntmachung des Hinweises auf die Patenterteilung: **04.07.2012**
(21) Anmeldenummer: 99117238.8
(22) Anmeldetag: 02.09.1999
(51) Int. Cl.: A61K 47/22, G01N 33/15, A61K 9/14

(54) **Charakterisierung der Verträglichkeit von biologisch aktiven Substanzen mit Polyvinylpyrrolidonen**
Determining the compatibility of biologically active materials with polyvinylpyrrolidones
Détermination de la compatibilité de matériaux biologiquement actifs avec les polyvinylpyrrolidones

(30) Priorität: 18.09.1998 DE 19842914
(43) Veröffentlichungstag der Anmeldung: 22.03.2000
(73) Patentinhaber: Abbott GmbH & Co. KG, 65205 Wiesbaden (DE)
(72) Erfinder: Breitenbach, Jörg, Dr., 68199 Mannheim (DE); Heger, Robert, Dr., 69124 Heidelberg (DE); Simon, Dirk, Dr., 67112 Mutterstadt (DE); Liepold, Bernd, Dr., 68161 Mannheim (DE)
(74) Vertreter: Riedl, Peter

(56) Entgegenhaltungen:
- DE-A- 19 641 437
- J. BREITENBACH ET AL.: "Confocal Raman-spectroscopy: analytical approach to solid dispersions and mapping of drugs" PHARMACEUTICAL RESEARCH, Bd. 16, Nr. 07, Juli 1999 (1999-07), Seiten 1109-1113, XP008015910 New-York (US)
- A. T. M. SERAJUDDIN: "Solid dispersion of poorly water-soluble drugs: early promises, subsequent problems, and recent breakthroughs" JOURNAL OF PHARMACEUTICAL SCIENCES, Bd. 88, Nr. 10, Oktober 1999 (1999-10), Seiten 1058-1066, XP000851882 Washington (US)

## Beschreibung

Die vorliegende Erfindung betrifft ein Verfahren zur Charakterisierung der Verträglichkeit von biologisch aktiven Substanzen mit Polyvinylpyrrolidonen in einer Feststoffdispersion gemäß Anspruch 1. Weiterhin betrifft die Erfindung die Verwendung von 1,3-Bis-(pyrrolidon-1-yl)-butan als Dispergier- oder Lösungsmittel in einem Testsystem zur vorgenammten Verträglichkeitscharakterisierung.

Feststoffdispersionen, also homogene feinstdisperse Phasen von zwei oder mehreren Feststoffen sowie ihr Sonderfall der sogenannten "festen Lösungen" (molekulardisperse Systeme), sowie ihr Einsatz in der pharmazeutischen Technologie sind allgemein bekannt (vgl. Chiou und Riegelman, J. Pharm. Sci., 60, 1281-1300 (1971)).

Die Herstellung von festen Lösungen kann mit Hilfe von Schmelzeverfahren oder nach dem Lösungsverfahren erfolgen.

Als polymerer Hilfsstoff für die Herstellung solcher Feststoffdispersionen bzw. fester Lösungen eignet sich insbesondere Polyvinylpyrrolidon (PVP). Besonders vorteilhaft lassen sich auf PVP basierende feste Lösungen von biologisch aktiven Substanzen durch Schmelzextrusion herstellen, wie beispielsweise in der EP-A 240904 beschrieben ist.

Die Herstellung von Schmelzextrudaten stellt jedoch Mindestanforderungen an die eingesetzten Mengen. Stehen nur kleinere Wirkstoffmengen zur Verfügung, so läßt sich nicht sicher voraussagen, ob ein Wirkstoff zusammen mit PVP, sei es in der Schmelze oder in Co-präzipitaten, feste Lösungen bilden wird. Gerade bei der Entwicklung von auf neuen Wirkstoffen basierenden Arzneimitteln stehen jedoch häufig nur kleinere Mengen des Wirkstoffs zur Verfügung, so daß die Möglichkeit einer Vorhersage mit Hilfe eines einfachen Testsystems ausserordentlich wünschenswert erscheint.

Ebenfalls wünschenswert ist die Möglichkeit, hinsichtlich der Stabilität von festen Lösungen oder Feststoffdispersionen Vorhersagen treffen zu können. In Abhängigkeit von der Kompatibilität von Wirkstoff und PVP kann es nämlich zur Entmischung der vorher homogen dispersen Phase oder zur Rekristallisation des Wirkstoffs kommen. Eine solche Phasenseparation oder Rekristallisation ist natürlich wegen der damit verbundenen Änderung der Homogenität und des Freisetzungsverhaltens unerwünscht.

Aufgabe der vorliegenden Erfindung war es, auf einfache Weise die Charakterisierung der Verträglichkeit von biologisch aktiven Stoffen und PVP in einer Feststoffdispersion zu ermöglichen.

Demgemäß wurde ein Verfahren zur Charakterisierung der Verträglichkeit von biologisch aktiven Stoffen mit Polyvinylpyrrolidonen in einer Feststoffdispersion gefunden, welches dadurch gekennzeichnet ist, daß man die biologisch aktive Substanz in 1,3-Bis- (pyrrolidon-1-yl)-butan dispergiert oder löst und die entstehende Dispersion oder Lösung spektroskopisch beurteilt.

Das erfindungsgemäß verwendete Testsystem besteht nun zum einen aus einer oder mehreren biologisch aktiven Substanzen, zum anderen aus 1,3-Bis-(pyrrolidon-1-yl)-butan als Lösungs- oder Dispergiermittel.

Die Herstellung von 1,3-Bis-(pyrrolidon-1-yl)-butan ist an sich bekannt. So beschreibt zum Beispiel Breitenbach et al., Naturwissenschaften 42, 1955, 155;440, die Dimerisierung von N-Vinylpyrrolidon unter sauren Reaktionsbedingungen und die nachfolgende Hydrierung des so erhaltenen 1,3-Bis-(pyrrolidonyl)-1-butens zum 1,3-Bis-(pyrrolidon-1-yl)-butan. 1,3-bis-(pyrrolidon-1-yl)-butan stellt eine ölige, farblose Flüssigkeit mit einem Siedepunkt von 205-215°C (0,2 mbar) dar. Aus der DE-A 196 41 437 ist bekannt, daß sich 1,3-Bis-(pyrrolidon-1-yl)-butan (im weiteren auch DHVP genannt) als Lösungsmittel oder Solubilisator für kosmetische oder pharmazeutische Wirkstoffe eignet.

Das Testsystem eignet sich prinzipiell für alle pharmazeutischen Wirkstoffe, Pflanzenschutzmittel, Nahrungsergänzungsmittel oder kosmetische Wirkstoffe. Weiterhin lassen sich auch Waschmittelenzyme auf ihre Verträglichkeit mit PVP untersuchen. Als Polyvinylpyrrolidone (PVP) kommen alle PVP-Arten mit K-Werten nach Fikentscher von 10 bis 110 in Betracht.

Verträglichkeit oder Kompatibilität im Sinne dieser Erfindung bezeichnet die Tendenz einer Substanz, mit PVP eine homogene, stabile Feststoffdispersion auszubilden, wobei diese Feststoffdispersion insbesondere eine feste Lösung, also eine molekulardisperse Verteilung der Komponenten ineinander, darstellt.

Zur Durchführung des erfindungsgemäßen Verfahrens wird eine Lösung oder Dispersion des Wirkstoffs oder von Wirkstoffmischungen in DHVP hergestellt. Die Mengenverhältnisse sind im Prinzip frei wählbar und hängen naturgemäß wesentlich von den Eigenschaften des Wirkstoffs ab. Es empfiehlt sich jedoch, die Konzentrationsbereiche in dem Testsystem so zu wählen, daß sie den für Extrudatformenen typischen Wirkstoffgehalten entsprechen, also im allgemeinen 0,1 bis 70, bevorzugt 10 bis 30 Gew.-% an Wirkstoff, bezogen auf das Gesamtgewicht des Testsystems. Im Einzelfall können aber auch 60 gew.-%ige oder sogar höher konzentrierte Lösungen oder Dispersionen hergestellt werden. Bevorzugt wird man Testreihen mit ansteigenden Wirkstoffgehalten durchführen. Der oder die Wirkstoffe werden eingewogen, mit den entsprechenden Mengen an DHVP versetzt, und vorzugsweise gerührt, üblicherweise mit einem typischen Labormagnetrührer bei 5 bis 200 Upm. Löst sich eine Substanz nicht bei Raumtemperatur, so kann man das Testsystem auch erwärmen. Bevorzugt erfolgt das Erwärmen so, daß es in etwa der Aufheizrate in einer späteren Schmelzeformulierung entspricht, also mit 0,5 bis 5°C/min. Vorzugsweise wird das Testsystem auf maximal 140°C erwärmt. Im Einzelfall kann man aber auch bis zum Siedepunkt des DHVP erwärmen. Anschließend läßt man das Testsystem auf Raumtemperatur abkühlen.

Die fertigen Testsysteme werden spektroskopisch begutachtet. Vorzugsweise erfolgt dies visuell mit einem Mikroskop, beispielsweise mit einem üblichen Labormikroskop mit einer Auflösung von 0,04 n.A.. So wird festgestellt, ob sich klare Lösungen oder Dispersionen bilden. Weiterhin wird das Rekristallisationsverhalten begutachtet. Vor allem bei Testsystemen, in denen der Wirkstoff durch Erwärmen in Lösung gebracht wurde, stellt das Rekristallisationsverhalten direkt nach dem Abkühlen auf Raumtemperatur ein wichtiges Kriterium dar. Testsysteme, in denen der Wirkstoff nicht direkt nach Beendigung des Rührens bzw. nach Abkühlen rekristallisiert, werden auf Langzeitstabilität untersucht:
- Stehenlassen bei Raumtemperatur für 24 Stunden
- nach 1 Monat, 3 Monaten, 6 Monaten bei Lagerung in Klimazone 2 (25°C, 60 % relative Luftfeuchte) und Klimazone 4 (20°C, 70 % relative Luftfeuchte)
- Stresslagerung bei 40°C, 75 % relative Luftfeuchte von bis zu 6 Monaten.

Untersucht wird stets, ob der Wirkstoff aus der Lösung rekristallisiert.

Weiterhin kann man die Testsysteme auch mit Hilfe der konfokalen Ramanspektroskopie auf ihren amorphen Charakter untersuchen.

Ebenso eignet sich die Methode der Differential Scanning Calorimetry.

Es hat sich gezeigt, daß sich aus dem Rekristallisationsverhalten aus einer DHVP-Lösung direkte Rückschlüsse auf die Stabilität einer durch Schmelzextrusion oder durch Co-präzipitation erhaltenen festen Lösung eines Wirkstoffs mit Polyvinylpyrrolidonen ziehen lassen. Es besteht eine gute Korrelation zwischen der Stabilität einer Lösung des Wirkstoffs in DHVP und der Stabilität eines wirkstoffhaltigen PVP-Schmelzextrudats oder Co-präzipitats. Weiterhin läßt auch das Auflösungsverhalten des Wirkstoffs in DHVP direkte Rückschlüsse auf die Neigung einer Substanz, mit PVP feste Lösungen zu bilden, zu, und zwar sowohl hinsichtlich eines geeigneten Temperaturprofils bei Schmelzprozessen wie der Extrusion als auch der einarbeitbaren Wirkstoffmenge.

Damit ermöglicht das Testsystem auf einfache Weise die Vorhersage der Verträglichkeit von biologisch aktiven Substanzen mit Polyvinylpyrrolidonen, insbesondere von durch Schmelzeverfahren erhältlichen Produkten.

Untersucht wurde beispielsweise das Auflösungsverhalten von Acetylcystein, Acetylsalicylsäure, Anipamil-HCl, Bentazon, Benzocain, Bezafibrat, Biperiden, Butazolidin, Captopril, Carbamazepin, Chloramphenicol, Cromoglicinsäure, Clotrimazol, Coffein, Cyclosporin, Diazepam, Diclofenac-natriumsalz, Dilliazon, Diltiazem, Dimetridazol, Diphenhydramin-HCl, 5,5-Diphenyl-hydantoin, Erythromycin-stearat, Esupron, Fenofibrat, Flecainid, Furosemid, Fluconazol, Gallopamil-HCl, Glibenclamid, Hydrochlorothiazid, Ibuprofen, Indometazin, Itraconazol, Ketoprofen, Melperon, Metazachlor, Nalixidinsäure, Naphthidrofuryl, Nexopamil, Nifedipin, Nitrendipin, Nitrofurantoin, Oxybutyrin, Paracetamol, Pentoifyllin, Paroxetin, Prazosin, Propafenon-HCl, Pseudoephedrin-HCl, Ranitin-HCl, Riboflavin, Selegilin-HCl, Sulfamethazin, Sulfamethoxazol, Sulfathiazol, Theophyllin, Tolbutamid, Triamteren, Trimethoprim, Zotepin.

In der nachstehenden Tabelle sind die Ergebnisse von Lösungsversuchen bei zwei unterschiedlichen Konzentrationen und der Vergleich mit den entsprechenden Schmelzextrudaten mit PVP K 30.

**Tabelle**

| | 10 gew.-%ig in DHVP | 30 gew.-%ig in DHVP | Extrudat 30 gew.-%ig in PVP |
|---|---|---|---|
| | RT: + | RT: - | |
| Esupron | Rekrist.: - | Lsg. bei 70°C | + |
| | | Rekrist. n. 20 h | |
| | RT: + | RT: - | |
| Gallopamil-HCl | Lsg. bei 60°C | Lsg. bei 60°C | + |
| | Rekrist.: - | Rekrist. n. 96 h | |
| | RT: + | RT: + | + |
| Ibuprofen | Rekrist.: - | Rekrist.: - | |
| | RT: - | RT: - | |
| Nifedipin | Lsg. bei 50°C | Lsg. bei 50°C | + |
| | Rekrist.: - | Rekrist. n. 24 h | |
| | RT: - | RT: - | |
| Paracetamol | Lsg. bei 90°C | Lsg. bei 90°C | + |
| | Rekrist.: - | Rekrist.: - | |
| | RT: + | RT: - | |
| Zotepin | Rekrist.: - | Lsg. bei 70°C | + |
| | | Rekrist. n. 40 h | |

| | | | |
|---|---|---|---|
| RT: Raumtemperatur +: Lösung (fest oder flüssig) -: keine Lösung | | | |

Alle oben aufgeführten Wirkstoffe, die bei Extrusion feste Lösungen bilden, lösen sich auch in DHVP im für die Extrusion typischen Temperaturbereich zwischen Raumtemperatur und 140°C.

Zum Vergleich:
Theophyllin
unlöslich in DHVP, keine feste Lösung mit PVP
löslich in DMSO (Dimethylsulfoxid)

Ibuprofen
löslich in DHVP, feste Lösung mit PVP
löslich in DMSO

Während für das DHVP-Testsystem eine Korrelation zwischen Löslichkeit und der Bildung fester Lösungen besteht, ist für DMSO keine solche Korrelation gegeben.

## Patentansprüche

1. Verfahren zur Charakterisierung der Verträglichkeit von biologisch aktiven Substanzen mit Polyvinylpyrrolidonen in einer Feststoffdispersion, **dadurch gekennzeichnet, dass** man eine oder mehrere biologisch aktive Substanzen in 1,3-Bis-(pyrrolidon-1-yl)-butan löst oder dispergiert und die entstehende Lösung oder Dispersion spektroskopisch beurteilt.

2. Verfahren nach Anspruch 1, **dadurch gekennzeichnet, dass** man die Mischung aus biologisch aktiver Substanz und 1,3-Bis-(pyrrolidon-1-yl)-butan auf bis zu 140°C erwärmt.

3. Verwendung von 1,3-Bis-(pyrrolidon-1-yl)-butan als Dispergier- oder Lösungsmittel in einem Testsystem zur Charakterisierung der Verträglichkeit von biologisch aktiven Substanzen mit Polyvinylpyrrolidonen in einer Feststoffdispersion, wobei das Testsystem aus einer oder mehreren biologisch aktiven Substanzen und 1,3-Bis-(pyrrolidon-1-yl)-butan besteht und optional Formulierungshilfsmittel enthalt.

4. Verwendung nach Anspruch 3, wobei das Testsystem 10 bis 70 Gew.-% an biologisch aktiver Substanz enthält.

## Claims

1. A method for characterizing the compatibility of biologically active substances with polyvinylpyrrolidones in a solid dispersion, **characterized in that** one or more biologically active substances are dissolved or dispersed in 1,3-bis-(pyrrolidone-1-yl) butane and the resulting solution or dispersion is assessed spectroscopically.

2. The method according to claim 1, **characterized in that** the mixture of biologically active substance and 1,3-bis-(pyrrolidone-1-yl)-butane is heated up to a maximum of 140°C.

3. The use of 1,3-bis-(pyrrolidone-1-yl)-butane as a dispersant or solvent in a test system to characterize the compatibility of biologically active substances with polyvinylpyrrolidones in a solid dispersion, wherein the test system comprises one or more biologically active substances and 1,3-bis-(pyrrolidone-1-yl)-butane and optionally contains formulation agents.

4. The use according to claim 3, wherein the test system contains from 10 to 70 % by weight of biologically active substance.

## Revendications

1. Procédé pour la détermination de la compatibilité de substances biologiquement actives avec des polyvinylpyrrolidones dans une dispersion de matières solides, **caractérisé en ce que** l'on dissout ou disperse une ou plusieurs substances biologiquement actives dans du 1,3-bis-(pyrrolidon-1-yl)-butane et on évalue la solution ou la dispersion produite par spectroscopie.

2. Procédé selon la revendication 1, **caractérisé en ce que** l'on chauffe le mélange constitué d'une substance biologiquement active et de 1,3-bis-(pyrrolidon-1-yl)-butane jusqu'à 140°C.

3. Utilisation de 1,3-bis-(pyrrolidon-1-yl)-butane comme agent de dispersion ou de dissolution dans un système de test destiné à la détermination de la compatibilité de substances biologiquement actives avec des polyvinylpyrrolidones dans une dispersion de matières solides, le système de test étant constitué d'une ou plusieurs substances biologiquement actives et de 1,3-bis-(pyrrolidon-1-yl)-butane et contenant des auxiliaires de formulation éventuels.

4. Utilisation selon la revendication 3, dans laquelle le système de test contient de 10 à 70 % en masse de substance biologiquement active.
